Europäisches Patentamt

European Patent Office

Office européen des brevets

(11) Veröffentlichungsnummer: **0 378 137**
**A2**

(12) **EUROPÄISCHE PATENTANMELDUNG**

(21) Anmeldenummer: **90100273.3**

(22) Anmeldetag: **08.01.90**

(51) Int. Cl.5: **A61K 9/52**

(30) Priorität: **13.01.89 DE 3900811**

(43) Veröffentlichungstag der Anmeldung:
**18.07.90 Patentblatt 90/29**

(84) Benannte Vertragsstaaten:
**AT BE CH DE DK ES FR GB GR IT LI LU NL SE**

(71) Anmelder: **Kali-Chemie Pharma GmbH**
**Hans-Böckler-Allee 20 Postfach 220**
**D-3000 Hannover 1(DE)**

(72) Erfinder: **Block, Jürgen**
**Falterweg 4**
**D-3008 Schloss Ricklingen(DE)**
Erfinder: **Cassiere, Agnes**
**69 Hameau de Bois Préau 58-60 Route de**
**l'Empereur**
**F-92500 Reuill-Malmaison(FR)**
Erfinder: **Christen, Marie-Odile**
**27 avenue Marceau**
**F-75016 Paris(FR)**

(74) Vertreter: **Lauer, Dieter, Dr.**
**c/o Kali-Chemie Aktiengesellschaft Postfach**
**220**
**D-3000 Hannover 1(DE)**

(54) **Neue Arzneiform.**

(57) Es werden Arzneimittelzubereitungen beschrieben, welche es ermöglichen auch Wirkstoffe mit organoleptisch ungünstigen Eigenschaften in flüssiger Form oral zu applizieren. Diese enthalten einen pharmazeutischen Wirkstoff enthaltende filmbeschichtete Partikel, deren wirkstoffhaltige Partikelkerne mit einem Film, welcher aus einem Gemisch aus einem physiologisch verträglichen, in Wasser unlöslichen polymeren Filmbildner und einem physiologisch verträglichen, in Wasser nur bei pH-Werten von $\leq 5$ löslichen polymeren Filmbildner und gegebenenfalls weiteren physiologisch verträglichen Hilfsstoffen gebildet ist, überzogen sind. Die Partikel sind zusammen mit physiologisch verträglichen polymeren Suspendiermitteln und gegebenenfalls weiteren physiologisch verträglichen pharmazeutischen Hilfsstoffen in in Wasser aufsuspendierbaren Gemengen enthalten.

## Neue Arzneiform

Die vorliegende Erfindung betrifft neue Arzneimittelzubereitungen, welche es ermöglichen auch pharmazeutische Wirkstoffe, welche unangenehme organoleptische Eigenschaften, z.B. einen durchdringenden äußerst schlechten Geschmack, besitzen oder welche in wäßriger Lösung unbeständig sind, in flüssiger Form oral zu verabreichen.

Pharmakologische Wirkstoffe, welche einen schlechten Geschmack oder sonstige unangenehme organoleptische Eigenschaften besitzen, werden zur oralen Applikation im allgemeinen zur Kaschierung ihrer ungünstigen organoleptischen Eigenschaften in Form von mit einem Überzug aus Zucker und/oder polymeren Filmbildnern versehenen Dragees oder filmüberzogenen Tabletten verabreicht.

Derartige dragierte feste Arzneiformen haben den Nachteil, daß sie nicht teilbar sind und somit die durch den Wirkstoffgehalt des einzelnen Dragees vorgegebene Dosis nicht individuellen Erfordernissen angepaßt werden kann. Besonders in der Pädiatrie ist es wichtig, pharmazeutische Wirkstoffe je nach Alter und Krankheitszustand des zu behandelnden Kindes individuell zu variieren. Auch bei Erwachsenen, insbesondere älteren Patienten, kann das Bedürfnis nach individuell angepaßter Dosierung bestehen. Ferner gibt es auch Patienten, welche mit dem Schlucken ungeteilter fester Arzneiformen Schwierigkeiten haben.

Es besteht daher ein Bedürfnis nach einer Arzneiform, in welcher auch Wirkstoffe mit unangenehmen organoleptischen Eigenschaften, insbesondere einem schlechten Geschmack, und/oder in wäßriger Lösung unbeständige Wirkstoffe in flüssiger individuell dosierbarer Form oral ohne Geschmacksbelästigung eingenommen werden können. Aufgabe der vorliegenden Erfindung ist es, eine Arzneiform zur Verfügung zu stellen, welche die Einnahme von Wirkstoffen mit organoleptisch unangenehmen Eigenschaften in flüssiger Form und individuell angepaßter Dosierung, jedoch unter Kaschierung der unangenehmen organoleptischen Eigenschaften, insbesondere eines schlechten Geschmacks, ermöglicht.

Erfindungsgemäß wird diese Aufgabe dadurch gelöst, daß der Wirkstoff in zur Herstellung von in Wasser aufsuspendierbaren pulver- oder granulatartigen Arzneimittelzubereitungen geeignete wirkstoffhaltige, filmbeschichtete Partikel formuliert wird, deren wirkstoffhaltige Partikelkerne mit einem Film, welcher aus einem Gemisch aus einem physiologisch verträglichen in Wasser unlöslichen polymeren Filmbildner und einem physiologisch verträglichen in Wasser nur bei pH-Werten von ≦ 5 löslichen polymeren Filmbildner, und gegebenenfalls weiteren physiologisch verträglichen Hilfsstoffen gebildet ist, überzogen sind. Zur Herstellung von in Wasser aufsuspendierbaren Arzneiformen werden die wirkstoffhaltigen, filmbeschichteten Partikel mit physiologisch verträglichen polymeren Suspendiermitteln und gegebenenfalls weiteren physiologisch verträglichen pharmazeutischen Hilfsstoffen zu erfindungsgemäßen in Wasser aufsuspendierbaren pulver- oder granulatartigen Gemengen verarbeitet.

Erfindungsgemäß werden in den filmbeschichteten Partikeln die Dicke des Films bzw. das Gewichtsverhältnis von wirkstoffhaltigem Partikelkern zu Filmüberzug und in dem Filmmaterial das Verhältnis von in Wasser unlöslichem Filmbildner zu in Wasser in saurem Medium löslichem Filmbildner so gewählt, daß die Partikel in Wasser mindestens 30 Minuten suspendiert werden können, ohne daß eine organoleptisch feststellbare Freigabe des Wirkstoffes erfolgt, und daß andererseits jedoch durch den Film keine Behinderung der Wirkstofffreisetzung im Magen-Darm-Trakt verursacht wird, sondern im Magensaft der Filmüberzug so schnell durchlässig wird, daß eine weitgehende Freigabe des Wirkstoffes innerhalb von höchstens 15 Minuten erfolgen kann. Der Filmüberzug ist also in neutraler wäßriger Lösung für einen Zeitraum von mindestens einer halben Stunde praktisch diffusionsdicht für den Wirkstoff, beeinträchtigt aber nicht dessen Bioverfügbarkeit nach der oralen Applikation.

Beispielsweise werden die Dicke des Films und das Verhältnis von in Wasser unlöslichem Filmbildner zu in Wasser in saurem Medium löslichem Filmbildner so gewählt, daß bei Suspendieren der Partikel in Wasser von neutralem pH-Wert, das ist einem pH-Wert von 6,0 - 7,5, bei einer Temperatur von 23 ° C nach 30 Minuten weniger als 10 %, vorzugsweise weniger als 5 % des Wirkstoffes in die wäßrige Lösung freigegeben werden, und daß bei Suspendieren der Partikel in künstlichem Magensaft *) bei einer Temperatur von 37 ° C nach 10 Minuten über 50 %, vorzugsweise mindestens 75 % des Wirkstoffes in die wäßrige Lösung freigegeben werden.

*) Zusammensetzung:

Natriumchlorid 2,0 g

0,7 %-ige Salzsäure 7,0 ml

destilliertes Wasser ad 100 ml

pH-Wert 1,2

Ferner werden die Dichte und Größe der Partikel so gewählt, daß die Zubereitung sich leicht in Wasser zu einer Suspension suspendieren läßt. Zweckmäßigerweise haben die einzelnen Partikel einen Durchmesser von höchstens ca. 1,5 mm. Beispielsweise kann der Partikeldurchmesser in Größenordnungen von 0,1 - 1,5 mm, vorzugsweise 0,2 - 0,8 mm, insbesondere 0,2 - 0,6 mm liegen. Das spezifische Gewicht der Partikel kann je nach Partikelgröße und -zusammensetzung variieren. Als zweckmäßig erweist sich ein spezifisches Gewicht im Bereich von 1,0 bis 1,7 g/cm$^3$ Die Dicke des Filmüberzuges kann je nach den chemisch-physialischen Eigenschaften der verwendeten Filmkomponenten und des wirkstoffhaltigen Partikelkerns sowie der Partikelgröße variieren.

Zweckmäßigerweise soll das Filmgewicht jedoch 6 % des Gesamtgewichtes der filmüberzogenen Partikel nicht unterschreiten und kann beispielsweise 6-18, vorzugsweise 8-17, insbesondere 8-14 % des Gesamtgewichtes betragen. Die Schichtdicke des Filmes liegt vorzugsweise zwischen 8 und 16 μm, insbesondere zwischen 8 und 13 μm.

Die erfindungsgemäße in Wasser aufsuspendierbare granulat-oder pulverartige Arzneizubereitung kann zur oralen Applikation kurz vor der Verabreichung zu einer trinkbaren Suspension aufsuspendiert werden, so daß eine Einnahme in flüssiger Form und individueller Dosierung unter Kaschierung ungünstiger organoleptischer Eigenschaften des Wirkstoffes und/oder dessen Schutz vor Kontakt mit Wasser möglich ist.

Gewünschtenfalls können die erfindungsgemäßen wirkstoffhaltigen Partikel auch mit üblichen pharmazeutischen Träger- und Hilfsstoffen zu direkt als Trockenpulver oder Trockengranulat verzehrbaren Gemischen verarbeitet werden.

Die neuen zur Herstellung von wäßrigen Trinksuspensionen geeigneten Arzneiformen eignen sich insbesondere zur oralen Verabreichung von frisch vor der Verabreichung aus dem trockenen Pulver oder Granulat hergestellten Trinksuspensionen von solchen Wirkstoffen, die unvorteilhafte organoleptische Eigenschaften, insbesondere einen schlechten Geschmack oder Geruch entwickeln, sowie von solchen Wirkstoffen, welche in wäßriger Lösung instabil und/oder per se nicht löslich oder suspendierbar sind. Beispiele von Wirkstoffen, deren schlechter Geschmack einer oralen Verabreichung in üblichen Flüssigformen entgegensteht, sind schlechtschmeckende Terpenderivate, insbesondere quartäre Salze von Terpenaminoätherderivaten wie Pinaveriumsalze; Antiemetica, beispielsweise mehrfach basisch substituierte Benzamide wie Metoclopramid (= Paspertin[R]); schwefelhaltige Choleretica, beispielsweise Trithionderivate wie Anetholtrithion (= Sulfarlem[R]); bittere Chinolinderivate, beispielsweise Chinolinalkaloide wie Chinin und Chinidin und 8-Aminochinolinderivate wie Chloroquin; spasmolytisch wirksame Atropin- und Scopolaminderivate, beispielsweise N-Niederalkylscopolamine wie N-Butylscopolamin; Antibiotica z.B. vom Typ der Penicilline und Tetracycline, für ihren bitteren Geschmack bekannte Cefuroximderivate, insbesondere Cefuroximacetoxyethylester, und Chloramphenicol; Sulfonamide; in wäßriger Lösung nicht über lange Zeit ausreichend stabile Wirkstoffe, beispielsweise als Broncholytica und Antiasthmatica bekannte Phenylpropanolaminderivate wie Salbutamol; aber auch Enzyme wie beispielsweise Pankreatin.

Als besonders geeignet erweisen sich die neuen Arzneiformen für Pinaveriumsalze, insbesondere Halogenide wie Pinaveriumbromid (Dicetel[R]).

Der wirkstoffhaltige Partikelkern kann den Wirkstoff als solchen oder in mikroverkapselter Form allein, z.B. in Form größerer Wirkstoffkristalle oder kleiner Wirkstofftröpfchen, oder gemischt mit üblichen Tablettierungshilfsstof fen wie neutralen Trägerstoffen und gewünschtenfalls weiteren Zusätzen wie Bindemitteln, Schmier- und Gleitmitteln enthalten. Als Trägerstoffe eignen sich vorwiegend wasserlösliche oder in Wasser dispergierbare, bzw. in Magensaft lösliche oder dispergierbare Füll- und Trägerstoffe. Beispiele von in Wasser löslichen und dispergierbaren Füll- und Trägerstoffen sind u.a. Zucker wie z.B. Lactose oder Saccharose, Zuckeralkohole wie z.B. Mannit, Polyvinylpyrrolidon, Mischpolymerisate aus Vinylpyrrolidon/Vinylacetat, feste Polyäthylenglycole, mikrokristalline Zellulose, Stärke, Calciumphosphate und/oder Talkum. Als weitere Hilfsstoffe können in den Partikelkernen beispielsweise Bindemittel wie Celluloseätherderivate, z.B. Carboxymethylcellulose oder Methylcellulose, Stärkekleister, Polyvinylpyrrolidon, Gummi arabicum oder Gelatine Gleit- und Schmiermittel wie Talkum, hochdisperse Kieselsäure, Magnesiumstearat usw. enthalten sein.

Die Herstellung von Wirkstoff und pharmazeutische Hilfsstoffe enthaltenden Partikelkernen kann auf an sich bekannte Weise erfolgen. Beispielsweise können Wirk- und Hilfsstoffe auf an sich bekannte Weise gemischt und die Mischung gegebenenfalls zu einem als Partikelkern geeignetem Granulat bzw. Pellets granuliert oder pelletiert werden, oder gegebenenfalls nach Feucht- oder Trockengranulation zu geeigneten Formkörpern verpreßt werden.

Nach einer weiteren Ausführungsform kann die Herstellung wirkstoffhaltiger Partikelkerne auch durch Tränken von auf an sich bekannte Weise hergestellten Trägerstoffglobuli oder -pellets mit einer Wirkstofflösung oder durch Beschichten solcher Trägerstoffglobuli oder -pellets mit einer wirkstoffhaltigen Suspension erfolgen. Die Trägerstoffglobuli oder -pellets können aus an sich bekannten pharmazeutisch verträglichen festen anorganischen oder organischen Trägerstoffen gebildet sein. Als Beispiele von geeigneten Trägerstoffen seien genannt Zucker wie Lactose oder Saccharose, Calciumsalze wie Calciumsulfat oder Hydrogen-

3

phosphat, Kaolin, Polystryrolharze, mikrokristalline Zellulose und Stärke oder deren Gemische, insbesondere Zucker/Stärkegemische und Lactose/Cellulosegemische. Zur Herstellung von mit einer wirkstoffhaltigen Beschichtung versehenen Globuli können pharmazeutisch übliche Globuli mit einer Lösung oder Suspension des Wirkstoffes: welche vorteilhaft zusätzlich ein physiologisch verträgliches in Wasser oder Magensaft lösliches makromolekulares Klebemittel enthält, beschichtet werden. Die wirkstoffhaltige Beschichtung kann auf an sich bekannte Weise, z.B. durch Besprühen der Globuli mit einer das Beschichtungsmaterial gelöst und/ oder dispergiert in Wasser oder einem Gemisch aus Wasser und einem geeigneten organischen Lösungsmittel, beispielsweise einem niederen Alkohol und/oder Aceton, enthaltenden Flüssigkeit in Dragierkesseln oder einem Wirbelschichtbeschichter aufgebracht werden.

Als Klebemittel für die wirkstoffhaltige Beschichtung eignen sich beispielsweise in saurem wäßrigen Medium lösliche Acrylharze mit cationischem Charakter, beispielsweise Copolymerisate auf Basis von basischen und neutralen Acrylsäure- oder Methacrylsäureestern, insbesondere ein Acrylharz auf Basis von Dimethylaminoäthylmethacrylat und neutralen Methacrylsäureestern mit einem mittleren Molekulargewicht von 100.000 bis 200.000, vorzugsweise um 150.000. Ferner sind als Klebemittel wasserlösliche Hydroxypropylcellulose allein oder im Gemisch mit Acrylpolymerisaten oder Polyvinylpyrrolidone geeignet. Neben dem Wirkstoff und dem Klebemittel kann die wirkstoffhaltige Beschichtung noch weitere pharmazeutisch übliche Hilfsstoffe, z.B. wasserlösliche oder -dispergierbare organische und/oder anorganische Füllstoffe wie beispielsweise Zucker oder Zuckeralkohole oder Antiklebemittel wie Talkum enthalten. Falls die Freisetzung des Wirkstoffes erst im Darmbereich erfolgen soll, können die wirkstoffhaltigen Partikelkerne vor der erfindungsgemäßen Beschichtung vorab noch in an sich bekannter Weise mit einem magensaftresistenten Überzug aus physiologisch verträglichen, magensaftresistenten bei einem pH-Wert von über 5 löslichen Filmbildnern versehen werden.

Erfindungsgemäß sind die wirkstoffhaltigen Partikelkerne mit einem in neutraler wäßriger Lösung mindestens 30 min. lang für den Wirkstoff praktisch diffusionsdichten Film überzogen, welcher jedoch im Magensaft unverzüglich so porös wird, daß der Wirkstoff praktisch vollständig innerhalb von 15 Minuten freigegeben werden kann. Vorteilhafterweise werden bei Suspension der filmüberzogenen Partikel in künstlichem Magensaft bei 37 °C mindestens 75 % des Wirkstoffes innerhalb 10 Minuten freigesetzt und mindestens 90 % innerhalb 15 Minuten. Als für einen Wirkstoff "diffusionsdicht" wird im Rahmen der vorliegenden Erfindung ein Film bezeichnet, der beim Suspendieren der Partikel in neutralem Wasser eine Diffusion des Wirkstoffes aus dem Partikelkern in die umgebende Lösung so weitgehend verhindert, daß innerhalb von 30 Minuten höchstens 10 % des Wirkstoffes aus dem Partikelkern diffundieren. Vorzugsweise ist der Film so diffusionsdicht, daß höchstens 5 % zweckmäßigerweise nicht über 3 % des Wirkstoffes innerhalb 30 Minuten diffundieren.

Der diffusionsdichte Filmüberzug enthält erfindungsgemäß ein Gemisch aus in Wasser praktisch unlöslichen Filmbildnern und in Wasser bei einem pH-Wert von unter 5 bzw. in künstlichem Magensaft löslichen Filmbildnern, welche bei neutralem pH-Wert in Wasser ebenfalls praktisch nicht löslich und nur soweit quellbar sind, daß eine Diffusion des Wirkstoffes aus dem Partikelkern von in neutralem Wasser suspendierten Partikeln weitgehend verhindert ist und während 30 min. 10 %, vorzugsweise 5 %, nicht überschreitet. Zweckmäßig liegt die Diffusion des Wirkstoffes in den ersten 15 Minuten unter 3 %.

Als in künstlichem Magensaft lösliche Filmbildner können im Rahmen der vorliegenden Erfindung alle physiologisch verträglichen Filmbildner verwendet werden, welche sich im Verdauungstrakt bei einem pH-Wert unter 5 lösen können. Physiologisch verträgliche magensaftlösliche makropolymere Substanzen, welche in der pharmazeutischen Technik zur Herstellung von magensaftlöslichen nicht retardierenden Tablettenüberzügen Verwendung finden können, sind dem Fachmann allgemein bekannt. So werden zum Beispiel in Pharmazeutische Technologie von H. Sucker et al, 1. Auf lage, Thieme-Verlag, Stuttgart, New York 1978, in Lehrbuch der pharmazeutischen Technologie von R. Voigt, 5. Auflage, Verlag Chemie, Weinheim, 1984, und in Physikalische Chemie von A. Martin et al, 3. von H. Stricker überarbeitete Auflage, Wissenschaftliche Verlagsgesellschaft Stuttgart, 1987, als Filmbildner zur Herstellung von nichtretardierenden Überzügen für Tabletten und Dragees geeignete polymere Filmbildner aufgeführt. U.a. sind geeignet in Wasser nur bei pH-Werten <5 lösliche kationische Acrylharze oder Hydroxypropylmethylzellulosen sowie auch substituierte Aminomethylzelluloseäther wie Benzylamino- oder Diäthylaminomethylzellulosen.

Als im Rahmen der vorliegenden Erfindung besonders geeignet erweisen sich Acrylharze mit kationischem Charakter, z.B. Mischpolymerisate auf Basis von basisch substituierten Methacrylsäureestern und neutralen Methacrylsäureestern, insbesondere auf Basis von Dimethylaminoäthylmethacrylat und neutralen Methacrylsäureestern. Das mittlere Molekulargewicht solcher kationischer Acrylmischpolymerisate liegt vorzugsweise zwischen 100.000 und 200.000, insbesondere um 150.000.

Als wasserunlösliche polymere Filmbildner können alle physiologisch verträglichen wasserunlöslichen Filmbildner verwendet werden. Derartige Filmbildner sind in der pharmazeutischen Technik zur Herstellung von retardierenden Filmüberzügen für Tabletten und Dragees bekannt. So werden z.B. in den vorgenannten Lehrbüchern von K. H. Bauer et al und von R. Voigt u.a. aufgeführt: unlösliche Zelluloseätherderivate wie Äthylzellulose, Zelluloseesterderivate wie z.B. Zelluloseacetatbutyrat, Acrylharze wie Copolymerisate von Methacrylsäureestern mit Trimethylammonium-äthylmethacrylat-hydrochlorid oder Carboxyäthylmethacrylat, Schellack, Zein oder unlösliche Vinylpolymere wie Polyvinylacetat/phthalat.

Als im Rahmen der vorliegenden Erfindung besonders geeignet haben sich Äthylzellulose, insbesondere Äthylzellulose der Typen 6-10 cP, USP XXI, oder Schellack erwiesen.

Neben den vorgenannten Filmbildnern kann der Filmüberzug noch bis zu 40 % an weiteren wasserabweisenden Substanzen und pharmazeutisch üblichen Hilfsmitteln enthalten, beispielsweise physiologisch verträgliche hydrophobe Stoffe, wie Fette und fettähnliche Substanzen, z.B. durch niedere organische Säuren mono- oder diacylierte Monoglyceride höherer Fettsäuren, z.B. Mono- und Diacetate von Monoglyceriden, oder Wachse, z.B. Mischungen aus Carnaubawachs und Bienenwachs, Salze, insbesondere Erdalkalimetallsalze, höherer Fettsäuren wie z.B. Magnesiumstearat. Als weitere Hilfsmittel können Antiklebemittel wie z.B. Talkum, Farbstoffe usw. zugesetzt werden.

Der Anteil an magensaftlöslichen polymeren Filmbildnern in dem diffusionsdichten Film kann je nach den physikochemischen Eigenschaften der Filmkomponenten und der Filmdicke variieren. Außerdem können die zur Erreichung einer ausreichenden Diffusionsdichte günstige Filmdicke und -zusammensetzung auch je nach den chemophysikalischen Eigenschaften des eingesetzten Wirkstoffes und der gegebenenfalls in dem Partikelkern enthaltenden weiteren Hilfsstoffe variieren. Zweckmäßig beträgt die Menge an magensaftlöslichen Polymeren mindestens 20 % und höchstens 60 % des Gesamtgewichtes des Filmüberzugs und beträgt vorteilhaft zwischen 20 und 40 % des Gesamtgewichtes des Filmüberzugs. Das Verhältnis von magensaftlöslichen polymeren Filmbildnern zu wasserunlöslichen polymeren Filmbildnern kann je nach Art der physikochemischen Eigenschaften der Filmbildner und Art und Menge der sonstigen in dem Filmüberzug vorhandenen Hilfsstoffe variieren. Zweckmäßig kann das Verhältnis von 0,3 : 1 bis 2 : 1 betragen. Sofern in dem Film keine oder nur geringe Mengen an weiteren wasserabweisenden Hilfsstoffen vorhanden sind, kann das Verhältnis im unteren Teil des angegebenen Bereichs liegen. Bei An wesenheit von größeren Mengen, z.B. Mengen von zwischen 12 % und 25 % des Gesamtfilmgewichtes, an wasserabweisenden Hilfsstoffen, beispielsweise Magnesiumstearat und/oder fett- und wachsartigen Substanzen, ist ein entsprechend höheres Verhältnis von magensaftlöslichem Filmbildner zu wasserlöslichem Filmbildner zweckmäßig. Zum Beispiel haben sich in diesem Fall Verhältnisse von 0,8 : 1 bis 2 : 1, insbesondere 0,9 : 1 bis 1,8 : 1 als geeignet erwiesen.

Als besonders günstig hat sich ein Gemisch von polymeren Filmbildnern erwiesen, worin als magensaftlösliches Polymeres ein Copolymerisat auf Basis von Dimethylaminoäthylmethacrylat und neutralen Methacrylsäureestern mit einem Molekulargewicht von zwischen 100.000 und 200.000 und als wasserunlöslicher polymerer Filmbildner Äthylzellulose 6-10 cP, USP XXI oder Schellack verwendet werden.

Die wirkstoffhaltigen Partikel können auf an sich bekannte Weise mit dem diffusionsdichten Überzug versehen werden, indem man die Partikel mit einer die Filmbildner und gegebenenfalls weiteren Hilfsstoffe enthaltenen Lösung oder Suspension nach an sich bekannten Methoden befilmt. Das Aufbringen des Filmes kann z.B. erfolgen, indem man die Partikel mit einem Gemisch aus Wasser und einem niedrigen Alkohol, z.B. Isopropanol, welches die Filmbildner und gegebenenfalls weitere Hilfsstoffe gelöst und/oder suspendiert enthält, in einem Dragierkessel oder einem Wirbelschichtbett besprüht und die beschichteten Partikel gewünschtenfalls anschließend trocknet.

Neben den filmbeschichteten wirkstoffhaltigen Partikeln enthält das erfindungsgemäße in Wasser aufsuspendierbare Gemenge noch ein Suspendiermittel und gewünschtenfalls weitere übliche pharmazeutische Hilfsstoffe. Als Suspendiermittel können in der pharmazeutischen Technik als Suspendiermittel an sich bekannte physiologisch verträgliche in kaltem Wasser (das ist bei Raumtemperatur) kolloidal lösliche natürliche, halbsynthetische oder synthetische makromulekulare Stoffe verwendet werden. Als Beispiele geeigneter Suspendiermittel seien genannt wasserlösliche Zelluloseäther, z.B. verätherte Carboxymethylzellulosen, wasserlösliche Polyvinylalkohole, Pektine oder Alginate.

Als weitere Hilfsstoffe können noch in Wasser gegebenenfalls kolloidal lösliche Füll- und Verdickungsmittel und die Viskosität erhöhende Mittel, wie beispielsweise mehrwertige Zuckeralkohole, Kieselgele, anorganische Kolloide, oder mikrokristalline Zellulosen oder Zucker zugesetzt werden.

Die filmbeschichteten wirkstoffhaltigen Partikel können mit den übrigen Hilfsstoffen in an sich bekannter Weise gemischt werden. Die Mischung kann gewünschtenfalls in einer üblichen Einzeldosis entsprechenden Anteilen in Portionsbeutel abgepackt werden.

Die nachfolgenden Beispiele sollen die Erfindung näher erläutern, ohne jedoch ihren Umfang zu beschränken.

Beispiel 1:

Pinaveriumbromid-haltige filmbeschichtete Pellets

| I.) Pinaveriumbromid-haltige Pelletkerne | | |
|---|---|---|
| Zusammensetzung: | mg/1g Gesamtgew. | g/Ansatz |
| Pinaveriumbromid | 56,5 | 711,9* |
| Copolymerisat | 27,0 | (340,2) |
| Dimethylaminoäthylmethacrylat/Methacrylsäureester[1] | | |
| ber. als Trockensubstanz, | | |
| als 12,5 %-ige Lösung in Aceton/Isopropanol | - | 2721,6* |
| Hydroxypropylcellulose[2] | 2,5 | 31,5* |
| Talkum[3] | 8,0 | 100,8* |
| Zuckerkügelchen[4] (Durchmesser 0,425-0,5mm) | 790,6 | 9487,2 |
| Isopropanol | - | 1560 * |
| entmineralisiertes Wasser | | 684 * |
| Gesamt | 884,6 | 10.671.4 |

* einschließlich 5 % Sprühverlust

[1] = Acrylharz mit kationischen Charakter, mittleres Molekulargew. 150.000, Eudragit[R]E, Hersteller Röhm GmbH, Darmstadt

[2] = Handelsbezeichnung Klucel[R]LF wasserlöslich, Hersteller: Hercules Filter Corp.

[3] = Micro Talcum = Talkum Feinheitsgrad Mikro

[4] = handelsübliche Kügelchen aus einem Zucker/Stärke-Gemisch

Herstellung

Pinaveriumbromid und Talkum werden in einer Lösung aus Hydroxypropylcellulose in Isopropanol/Wasser mittels eines Ultrarührers (Ultra Turrax[R]) während 5 Min. homogen dispergiert. Anschließend wird die 12,5 %-ige Eudragit[R]-E-Lösung in die Suspension eingerührt.

Die Zuckerkügelchen werden in einem Wirbelschichtbeschichter (Typ WSLD 5 der Firma Glatt) durch Besprühen mit der so hergestellten wirkstoffhaltigen Suspension unter den folgenden Arbeitsbedingungen beschichtet:

Düsenquerschnitt : 3 x 1,2 mm

Temperaturführung: 30/33/35 °C

Luftführung: 140/160/125 m³/h

Sprührate : 0,05 l/Min.

Sprühdruck : 1,3 bar

Anschließend werden die wirkstoffhaltigen Pellets 6 Std. im Hordentrockenschrank bei 40 °C getrocknet und über ein 0,8 mm-Sieb gegeben.

| II.) Filmbeschichtete Pellets | | | |
|---|---|---|---|
| Zusammensetzung: | mg/1g Gesamtgew. | g/Ansatz | Gew.-% d.Filmes |
| Filmbestandteile | | | |
| a) Polymere Filmbildner | | | |
| 1. Copolymerisat Dimethylaminoäthylmethacrylat/Methacrylsäureester[1]*berechnet als Trockensubstanz (=TS), | 31,78 | [118,6*] | 27,5 |
| als 12,5%-ige Lösung in Aceton/Isopropanol | - | 948,8* | |
| 2. Ethylcellulose (7cP)[5] berechnet als TS, | 50,0 | [186,5*] | 43,3 |
| als 12,5%-ige Lösung in Isopropanol | - | 1492,0* | |
| b) Sonstige Wirkstoffe | | | |
| Talkum[3] | 16,30 | 60,8* | 14,2 |
| Magnesiumstearat | 17,31 | 64,6* | 15,0 |
| Wasser | - | 50,0* | |
| Isopropanol | - | 2275,0* | |
| insgesamt | 115,39 | | 100 |
| Pinaveriumbromid-haltige Pellets aus Stufe I | 884,61 | 2969,7 | 3000,0 |
| Gesamt | 1000 | 3400,5 | 3100 TS |

*einschließlich 10 % Sprühverlust

[1] = Acrylharz mit kationischem Charakter, mittleres Molekulargew. 150000, Eudragit[R]E, Hersteller Röhm GmbH, Darmstadt

[5] = Ethylcellulose 7cP, USP XXI (NF XVI)

[3] = Micro Talcum = Talkum Feinheitsgrad Mikro

Herstellung:

Talkum und Magnesiumstearat werden in 0,7 kg Isopropanol eingerührt und während 5 min mit einem Ultra-Turrax[R]-Rührer dispergiert. Sodann werden die 12,5%-ige Eudragit[R] E-lösung, die 12,5%-ige Ethylcelluloselösung und das restliche Isopropanol zugegeben und die Suspension wird weitere 2-3 Minuten dispergiert.

Die Pellets werden in einem Wirbelschichtgerät durch Besprühen mit der so hergestellten Suspension unter folgenden Arbeitsbedingungen mit dem Filmmaterial beschichtet:

Düsendurchmesser 1,8 mm

Düsenabstand 30,0 cm

Temperaturführung 31° C/33° C/36° C

Sprührate 0,045 l/min

Sprühdruck 1,5 bar

Luftführung 250/300/200 m³/h

Trockenzeit 120 sec.

Anschließend werden die filmbeschichteten Pellets in einem Horden-Trockenschrank 6 Std. bei 40° C getrocknet. Es werden 3,36 kg filmbeschichtete Pellets erhalten. Pellets mit einem Durchmesser von >0,8 mm werden abgesiebt.

Eine 50 mg Pinaveriumbromid entsprechende Menge der filmbeschichteten Pellets (~ 1 g) wurden in ein Gefäß mit 100 ml Wasser von 23 °C gegeben und gerührt. Nach 20 min. konnte kein bitterer Geschmack festgestellt werden.

Beispiel 2

Pinaveriumbromid-haltige filmbeschichtete Pellets

Die Pinaveriumbromid-haltigen Pelletkerne werden wie in Beispiel 1 beschrieben hergestellt und dann in der in Beispiel 1 beschriebenen Weise mit einem Film beschichtet.

Die Zusammensetzung der erhaltenen filmbeschichteten Pellets ist in der nachstehenden Tabelle 2 wiedergegeben.

Tabelle 2

**Beispiele 2A - 2G.**
Pinaveriumbromid-haltige filmbeschichtete Pellets

| Zusammensetzung in \ Bei mg/1g Gesamtmenge \spiel Nr. | 2 A | 2 B | 2 C | 2 D | 2 E | 2 F | 2 G |
|---|---|---|---|---|---|---|---|
| Pelletkerne enthaltend 5,5 Gew.-% Pinaveriumbromid | 884,6 | 884,6 | 884,6 | 893,5 | 920,0 | 836,4 | 911,2 |
| Filmmaterial insgesamt | 115,4 | 115,4 | 115,4 | 106,5 | 80,0 | 163,6 | 88,8 |
| Filmmaterialzusammensetzung in Gew. %<br>a) Filmbildende Polymere<br>  1. Copolymerisat Dimethyl-aminoäthylmethacrylat/Methacrylsäureester[1] | 40,9 | 37,5 | 27,5 | 20,2 | 37,5 | 37,5 | 31,3 |
|   2. Ethylcellulose (7cP)[3] | 36,4 | 33,4 | 43,3 | 56,3 | 33,4 | 33,4 | 31,3 |
| b) Sonstige Hilfsstoffe<br>  1) Talcum[3] | 14,0 | 16,1 | 14,2 | 23,5 | 14,1 | 14,1 | 12,4 |
|   2) Magnesiumstearat | | | 15,0 | | 15,0 | 15,0 | 25,0 |
|   3) Gemisch acylierter Monofettsäure-glycerinester[6] | | 13,0 | | | | | |
|   4) Bienenwachs, Carnauba-wachs und Schellack-haltiges Gemisch[7] | 8,7 | | | | | | |

[6] = Myvacete[*] = fettartiges Gemisch teilweise acetylierter Monofettsäureglycerinester, Hersteller: ..............

[7] = Capol[*]600 = wachsartiges Gemisch aus Schellack/Bienenwachs/Carnaubawachs/Ethanol 5,8:9,6:9:6:75

Beispiel 3

Bestimmung der Wirkstofffreisetzung aus den filmbeschichteten Pinaveriumbromid-haltigen Pellets der Beispiele 1 und 2 nach der Dissolution-Test-Methode der USP XXI, Apparatus 2 (÷ Paddle Method):

A) in neutralem Wasser bei Raumtemperatur:

Eine 50 mg Pinaveriumbromid entsprechende Menge (~ 1g) filmbeschichtete Pellets werden in ein 900 ml Wasser von 23 °C enthaltendes verschließbares Gefäß gegeben. Das Gefäß wird verschlossen und der Inhalt während 30 min mit einem Rührblattrührer mit einer Drehgeschwindigkeit von 120 U/min. gerührt. Anschließend wird eine Probe des Wassers entnommen, die darin gelöste Menge an Pinaveriumbromid bestimmt und daraus der % Anteil an aus den Pellets diffundiertem Pinaveriumbromid in Gew.-% berechnet.

Die Ergebnisse sind in Tabelle 3, Spalte A wiedergegeben.

B) in künstlichem Magensaft bei pH 1,2 und 37 °C:

Eine 50 mg Pinaveriumbromid entsprechende Menge filmbeschichteter Pellets werden in ein 900 ml künstlichen Magensaft (Zusammensetzung siehe Fußnote Seite 3) von einer Temperatur von 37 °C enthaltendes verschließbares Gefäß gegeben. Das Gefäß wir geschlossen und der Inhalt bei einer Temperatur von 37 °C 10 min mit einem Rührblattrührer mit einer Drehgeschwinigkeit von 120 U/min. gerührt. Anschließend wird der in dem künstlichen Magensaft gelöste Anteil des Pinaveriumbromids in Gew.-% bestimmt. Die Ergebnisse sind in Tabelle 3, Spalte B, wiedergegeben.

Tabelle 3

| Diffusion von Pinaveriumbromid aus den filmbeschichteten Pellets der Beispiele 1 und 2 | | |
|---|---|---|
| Beispiel Nr. | A % diffundiertes Pinaveriumbromid in neutralem Wasser bei 23 °C nach 30 min. | B % diffundiertes Pinaveriumbromid in künst- in künstlichem Magensaft bei 37 °C nach 10 min. |
| 1 | 1,7 | 99 |
| 2 A | 9,6 | 87 |
| 2 B | 8,7 | 94,6 |
| 2 C | 1,14 | 92,5 |
| 2 D | 5,0 | 75,2 |
| 2 E | 9,2 | > 80 |
| 2 F | 2,44 | > 95 |
| 2 G | 4,40 | > 95 |

Beispiel 4

Salbutamol-haltige filmbeschichtete Kügelchen

| I.) Salbutamol ( = 1-(4-Hydroxy-3-hydroxymethylphenyl)-2-tert. butylaminoethanol) enthaltende Kügelchen | | |
|---|---|---|
| Zusammensetzung: | mg/1g Gesamtgew. | g/Ansatz |
| Salbutamolsulfat | 10,0 | 42 * |
| Lactose D 80, gemahlen | 40,0 | 166 * |
| Talkum[3] | 9,2 | 38,64* |
| Hydroxypropylzellulose[2] | 3,0 | 12,6 * |
| Copolymerisat Dimethylaminoäthylmethacrylat/Methacrylsäureester[1] berechnet als TS, | 31,0 | [130,2 *] |
| als 12,5%-ige Lösung in Aceton/Isopropanol | - | 1041,6 * |
| Isopropanol | - | 816,0 * |
| Wasser | - | 90,6 * |
| Zuckerkügelchen[4] (Durchmesser 0 ,425-0,5 mm) | 791,61 | 3166,44 |
| Gesamt | 884,61 | 3557,88 |

*einschließlich 5 % Sprühverlust

EP 0 378 137 A2

Herstellung:

Die gemahlene Lactose, der Wirkstoff und Talkum werden in einer Lösung aus Hydroxypropylzellulose in Isopropanol/Wasser mittels eines Ultrarührers (Ultra-Turrax[R]) während 2-3 min homogen dispergiert. Sodann wird die 12,5%-ige Eudragit[R]-E-lösung in die Suspension eingerührt.

Die Zuckerkügelchen werden in einem Wirbelschichtbeschichter (Typ (WSLDS der Firma Glatt) durch Besprühen mit der so hergstellten wirkstoffhaltigen Suspension unter den folgenden Arbeitsbedingungen mit einer wirkstoffhaltigen Schicht versehen:

Düsenquerschnitt 3 x 1,0 mm
Temperaturführung 30/33/35 °C
Luftführung 150 /180/125 m³ /h
Sprührate 0,06 l/min
Sprühmenge 2,75 l
Sprühdruck 1,3 bar

Anschließend werden die wirkstoffhaltigen Pellets 8 Stunden im Hordentrockenschrank bei 45 °C getrocknet und dann über ein 0,8 mm Sieb gegeben.

| II.) Filmbeschichtete Kügelchen | | |
|---|---|---|
| Zusammensetzung | mg/1g Gesamtgew. | g Ansatz |
| Filmbestandteile | | |
| a) Polymere Filmbildner | | |
| 1) Copolymerisat Dimethylaminoäthylmethacrylat/Methacrylsäureester[1] berechnet als TS, | 43,27 | [190,4 *] |
| als 12,5 %-ige Lösung in Aceton/Isopropanol | - | 15230 * |
| 2) Ethylcellulose (7cP)[5] berechnet als TS, | 38,51 | [169,4* ] |
| als 12,5 %-ige Lösung in Isopropanol | - | 1355,6* |
| b) Sonstige Hilfsstoffe | | |
| Talkum | 16,3 | 71,7* |
| Magnesiumstearat | 17,31 | 76,2* |
| Wasser | - | 60,0* |
| Isopropanol | - | 2706,0* |
| Salbutamol-haltige Kügelchen aus Stufe I | 884,61 | 3538,4 |
| Gesamt | 1000,0 | 4046,1TS |

*einschließlich 10% Sprühverlust

Herstellung:

Talkum und Magnesiumstearat werden in 1 kg Isopropanol eingerührt und mit einem Ultra-Turrax[R]-Rührer 3-5 min lang dispergiert.

Sodann werden die 12,5%-ige Ethylzelluloselösung, die 12,5%-ige Eudragit[R]-E-lösung, das restliche Isopropanol und das Wasser zugegeben und die Suspension nochmals dispergiert.

Die wirkstoffhaltigen Kügelchen werden in einem Wirbelschichtgranulator durch Besprühen mit der hergestellten Suspension unter Rühren unter den folgenden Arbeitsbedingungen mit dem Filmmaterial beschichtet:

Düsendurchmesser 1,8 mm
Düsenabstand 30 cm

Temperaturführung 31/33/35 °C
Luftführung 300/375/250 m³/h
Sprühdruck 1,5 bar
Sprührate 0,08 l/min
Sprühmenge 6,85 l

Anschließend werden die filmbeschichteten Kügelchen in einem Hordentrockenschrank 6 Std. bei 40 °C nachgetrocknet. Dann werden Kügelchen mit einem Durchmesser >0,8 mm abgesiebt.

Die Diffusion des Wirkstoffs aus den Pellets wird nach der in Beispiel 3 beschriebenen Methode (Rührblattdrehgeschwindigkeit 50 U/min.) bestimmt Gew.-% diffundierter Wirkstoff in neutralem Wasser von 23 °C nach 30 min < 0.5 %

Gew.-% diffundierter Wirkstoff in künstlichem Magensaft von pH 1,2 und 37 °C nach 10 min 100 %.

Beispiel 5:

Metoclopramid-haltige filmbeschichtete Kügelchen

| I.) Metoclopramid [ = 2-Methoxy-4-amino-5-chlorbenzoesäure-N-(-N′-diäthylaminoäthyl)-amid-monohydrochlorid] enthaltende Kügelchen | | |
|---|---|---|
| Zusammensetzung | mg/1g Gesamtgew. | g/Ansatz |
| Metoclopramid . HCl | 85,0 | 446,25* |
| Polyvinylpyrrolidon 25[8] | 17,0 | 89,25* |
| Zuckerkügelchen[4] (Durchmesser 0,85-1,0 mm) | 798,0 | 3990.0 |
| Isopropanol | - | 1000,0 * |
| Wasser | - | 75,0 * |
| Gesamt | 900,0 | 4525,5 |

* einschließlich 5 % Sprühverlust
[8] = Handelsbezeichnung Kollidon 25, Hersteller BASF AG.

Herstellung

Der gemahlene Wirkstoff wird in einer Lösung aus Polyvinylpyrrolidon 25 in Isopropanol/Wasser mittels eines Ultrarührers (Ultra Turrax[R]) während 5 Min. homogen dispergiert.

Die Zuckerkügelchen werden in einem Wirbelschichtbeschichter (Typ WSLD5 der Firma Glatt) durch Besprühen mit der wirkstoffhaltigen Suspension unter folgenden Arbeitsbedingungen beschichtet:

Düsenquerschnitt : 3 x 1,0 mm
Temperaturführung: 38/40/44 °C
Luftführung : 160/180/125 m³/h
Sprührate : 55 g/min.
Sprühdruck : 1,3 bar

Anschließend werden die wirkstoffhaltigen Pellets 12 Std. im Hordentrockenschrank bei 40-45 °C getrocknet und dann über ein 1,25 mm-Sieb abgesiebt.

| II.) Filmbeschichtete Kügelchen | | |
|---|---|---|
| Zusammensetzung | mg/1g Gesamtgew. | g/Ansatz |
| Filmbestandteile | | |
| a) Polymere Filmbildner | | |
| 1. Copolymerisat Dimethylaminoäthylmethycrylat/Methacrylsäureester[1] berechnet als TS, | 45,1 | (198,44*) |
| als 12,5 %-ige Lösung in Aceton/Isopropanol | - | 1587,5 * |
| 2. Ethylcellulose (7cP)[5] berechnet als TS, | 22,6 | ( 99,44*) |
| als 12,5 %-ige Lösung in Isopropanol | - | 795,5 * |
| b) Sonstige Hilfsstoffe | | |
| Talkum[3] | 11,0 | 48,4 * |
| Magnesiumstearat | 21,3 | 93,7 * |
| Wasser | | 52,2 * |
| Isopropanol | | 235,00* |
| Metoclopramid-haltige Kügelchen aus Stufe I | 900,0 | 3600,00 |
| Gesamt (TS) | 1000,0 | 4039,98 |

* einschließlich 10 % Sprühverlust

## Herstellung

Talkum und Magnesiumstearat werden in 1 kg Isopropanol eingerührt und mit einem Ultrarührer (Ultra Turrax[R]) 3-5 min. langsam dispergiert. Anschließend werden die 12,5 %-ige Eudragit[R] E-Lösung, die 12,5 %-ige Ethylcelluloselösung, das restliche Isopropanol und das Wasser zugegeben und die Suspension erneut dispergiert.

Die wirkstoffhaltigen Kügelchen werden in einem Wirbelschichtgerät durch Besprühen mit der hergestellten Suspension unter Rühren unter den folgenden Arbeitsbedingungen mit dem Filmmaterial beschichtet:

Düsendurchmesser : 1 x 1,8 mm

Düsenabstand : 30 cm

Temperaturführung: 31/33/36 °C

Luftführung : 325/400/250 m³/h

Sprührate : 0,08 l/min.

Sprühdruck : 1,6 bar

Die filmbeschichteten Kügelchen werden in einem Hordentrockenschrank 6 Std. bei 40 °C nachgetrocknet und über ein 1,25 mm-Sieb abgesiebt.

Die Diffusion des Wirkstoffes aus den Pellets wird nach der Dissolution-Test-Methode der USP XXI, Apparatus 1, wie folgt bestimmt:

Eine 50 mg Metoclopramid entsprechende Menge (~ 1g) filmbeschichtete Pellets werden in ein an einem drehbaren Schaft befestigtes Stahldrahtkörbchen gegeben, das in ein 900 ml Flüssigkeit enthaltendes verschließbares Gefäß eingetaucht und darin mit einer Rotationsgeschwindigkeit von 50 U/min. gedreht wird. Anschließend wird eine Probe der Flüssigkeit entnommen, die darin gelöste Menge an Metoclopramid bestimmt und daraus der %-Anteil an aus den Pellets diffundiertem Wirkstoff in Gew.-% berechnet.

Gew.-% diffundierter Wirkstoff in neutralem Wasser von 23 °C nach 30 min.: 0,5 %.

Gew.-% diffundierter Wirkstoff in künstlichem Magensaft von pH 1,2 und 37 °C nach 10 min.: 100,7 %.

## Beispiel 6:

14

Zu einer Trinksuspension aufbereitbares Pinaveriumbromidhaltiges Gemenge.

| Zusammensetzung: | |
|---|---|
| Pinaveriumbromid-haltige filmbeschichtete Pellets nach Beispiel 1 | 1,0 g |
| Sorbit | 0,5 g |
| Saccharose | 0,5 g |
| | 2,0 g |

Herstellung:

Die filmbeschichteten Pellets und die Hilfsstoffe werden auf an sich bekannte Weise gemischt und in Portionen von je 2 g in Portionsbeutel abgefüllt.

Ein Portionsbeutel dieses Gemenges in 100-200 ml Wasser angerührt ergibt eine trinkbare Suspension, welche frei von bitterem Geschmack ist.

Beispiel 7:

Zu einer Trinksuspension aufbereitbares Pinaverium-bromidhaltiges Gemenge

| Zusammensetzung: | |
|---|---|
| Filmbeschichtete Pinaverium-bromid-haltige Kügelchen gemäß Beispiel 1 | 1,0 g |
| mikrokristalline Zellulose[9] | 2,0 g |
| Saccharose | 7,5 g |
| | 10,5 g |

[9] Avicel[R] CL 611 Hersteller American Viscose Corporation

Herstellung:

Die filmbeschichteten Kügelchen werden auf an sich bekannte Weise gemischt und in Portionen von je 10,5 g in Portionsbeutel abgefüllt.

Der Inhalt eines Portionsbeutels in Wasser angerührt ergibt eine trinkbare Suspension, welche frei von bitterem Geschmack ist.

**Ansprüche**

1. Zur Herstellung von in Wasser aufsuspendierbaren Arzneimittelzubereitungen geeignete wirkstoffhaltige filmbeschichtete Partikel, dadurch gekennzeichnet, daß sie einen pharmazeutischen Wirkstoff in Form von wirkstoffhaltigen Partikelkernen enthalten, welche mit einem Film überzogen sind, welcher aus einem Gemisch aus einem physiologisch verträglichen, in Wasser unlöslichen polymeren Filmbildner und einem physiologisch verträglichen, in Wasser nur bei pH-Werten von $\leq 5$ löslichen polymeren Filmbildner und gegebenenfalls weiteren physiologisch verträglichen Hilfsstoffen gebildet ist.

2. Partikel nach Anspruch 1 dadurch gekennzeichnet, daß der Film eine solche Dicke und ein solches Verhältnis von in Wasser unlöslichem polymeren Filmbildner zu in Wasser bei pH-Werten von $\leq 5$ löslichem polymeren Filmbildner besitzt, daß die Partikel bei 30-minütigem Suspendieren in Wasser bei neutralem pH und einer Temperatur von 23 °C höchstens 5 % des Wirkstoffs in das Wasser abgeben und bei 10-minütigem Suspendieren in künstlichem Magensaft bei einer Temperatur von 37 °C mindestens 80

15

% des Wirkstoffes in die wäßrige Lösung freigeben.

3. Partikel nach Anspruch 2, dadurch gekennzeichnet, daß der Film eine Dicke von 8-16 μm besitzt.

4. Partikel nach Anspruch 2, dadurch gekennzeichnet, daß das Gewicht des Filmes 6-18 %, bezogen auf das Gesamtgewicht der filmbeschichteten Partikel beträgt.

5. Partikel nach Anspruch 1, dadurch gekennzeichnet, daß sie einen Durchmesser von höchstens 1,5 mm haben.

6. Partikel nach Anspruch 5, dadurch gekennzeichnet, daß sie einen Durchmesser von 0,2-0,6 mm haben.

7. Partikel nach Anspruch 1, dadurch gekennzeichnet, daß der Wirkstoff ein Wirkstoff aus der Gruppe Terpenaminoätherderivate, Antiemetica, insbesondere basisch substituierte Benzamidderivate, Choleretica, insbesondere Trithionderivate, Chinolinderivate, Spasmolytica, insbesondere Atropin- und Scopolaminderivate, Phenylpropanolaminderivate, Antibiotica und Enzyme ist.

8. Partikel nach Anspruch 7, dadurch gekennzeichnet, daß der Wirkstoff ein Pinaveriumsalz ist.

9. Partikel nach Anspruch 8, dadurch gekennzeichnet, daß der Wirkstoff Pinaveriumbromid ist.

10. Partikel nach Anspruch 1, dadurch gekennzeichnet, daß als in Wasser nur bei pH-Werten von ≤5 lösliche polymere Filmbilder Mischpolymerisate aus basisch substituierten Methacrylsäureestern und neutralen Methacrylsäureestern verwendet werden.

11. Partikel nach Anspruch 10, dadurch gekennzeichnet, daß der Filmbildner ein Mischpolymerisat aus Dimethylaminoäthylmethacrylat und neutralen Methacrylsäureestern mit einem mittleren Molekulargewicht zwischen 100.000 und 200.000 ist.

12. Partikel nach Anspruch 1, dadurch gekennzeichnet, daß als in Wasser unlösliche polymere Filmbildner wasserunlösliche Zellulosederivate oder Schellack verwendet werden.

13. Partikel nach Anspruch 12, dadurch gekennzeichnet, daß das wasserunlösliche Zellulosederivat eine Äthylzellulose ist.

14. Partikel nach Anspruch 1, dadurch gekennzeichnet, daß in dem Film ein Anteil von 20-40 % des Gesamtgewichtes des Filmes aus in Wasser nur bei pH-Werten ≤5 löslichen polymeren Filmbildnern besteht.

15. Partikel nach Anspruch 1, dadurch gekennzeichnet, daß in dem Film ein Gewichtsverhältnis von in Wasser nur bei pH-Werten ≤5 löslichen polymeren Filmbildnern zu in Wasser unlöslichen polymeren Filmbildnern von 0,3:1 bis 2:1 vorliegt.

16. Partikel nach Anspruch 1, dadurch gekennzeichnet, daß der Film zwischen 12 und 25 % seines Gesamtgewichtes an wasserabweisenden Hilfsstoffen enthält.

17. Partikel nach Anspruch 15, dadurch gekennzeichnet, daß in dem Film ein nur bei pH-Werten ≤5 in Wasser lösliches Copolymerisat aus Dimethylaminoäthylmethacrylat und neutralen Methacrylsäureestern mit einem mittleren Molekulargewicht zwischen 100.000 und 200.000 und eine in Wasser unlösliche Äthylzellulose und/oder Schellack vorliegen.

18. Partikel nach Anspruch 17, dadurch gekennzeichnet, daß der Film 12-25 % seines Gesamtgewichtes an wasserabweisenden Hilfsstoffen enthält und daß das Acrylatcopolymerisat und die Äthylzellulose und/oder Schellack in einem Gewichtsverhältnis von 0,8:1 bis 2:1 vorliegen.

19. Partikel nach Anspruch 7, dadurch gekennzeichnet, daß der Wirkstoff Metoclopramid ist.

20. Partikel nach Anspruch 7, dadurch gekennzeichnet, daß der Wirkstoff Salbutamol ist.

21. Arzneimittelzubereitung, dadurch gekennzeichnet, daß sie ein in Wasser aufsuspendierbares Gemenge darstellt, welches einen pharmazeutischen Wirkstoff enthaltende filmbeschichtete Partikel, deren wirkstoffhaltige Partikelkerne mit einem Film, welcher aus einem Gemisch aus einem physiologisch verträglichen, in Wasser unlöslichen polymeren Filmbildner und einem physiologisch verträglichen, in Wasser nur bei pH-Werten von ≤ 5 löslichen polymeren Filmbildner und gegebenenfalls weiteren physiologisch verträglichen Hilfsstoffen gebildet ist, überzogen sind, physiologisch verträgliche polymere Suspendiermittel und gegebenenfalls weitere physiologisch verträgliche pharmazeutische Hilfsstoffe enthält.

22. Arzneimittelzubereitung nach Anspruch 21, dadurch gekennzeichnet, daß in den wirkstoffhaltigen filmbeschichteten Partikeln eine solche Dicke des Filmes und in dem Film ein solches Verhältnis von in Wasser unlöslichem polymeren Filmbildner zu in Wasser bei pH-Werten von ≤ 5 löslichem polymeren Filmbildner vorliegen, daß die Partikel bei 30-minütigem Suspendieren in Wasser bei neutralem pH und einer Temperatur von 23 °C höchstens 10 % des Wirkstoffs in das Wasser abgeben und bei 10-minütigem Suspendieren in künstlichem Magensaft bei einer Temperatur von 37 °C mindestens 75 % des Wirkstoffes in die wäßrige Lösung freigeben.

23. Arzneimittelzubereitung nach Anspruch 22, dadurch gekennzeichnet, daß der Film eine Dicke von 8-16 μm besitzt

24. Arzneimittelzubereitung nach Anspruch 22, dadurch gekennzeichnet, daß das Gewicht des Filmes 6-18 %, bezogen auf das Gesamtgewicht der filmbeschichteten Partikel, beträgt.

25. Arzneimittelzubereitung nach Anspruch 21, dadurch gekennzeichnet, daß die wirkstoffhaltigen Partikel einen Durchmesser von höchstens 1,5 mm haben.

26. Arzneimittelzubereitung nach Anspruch 25, dadurch gekennzeichnet, daß die wirkstoffhaltigen Partikel einen Durchmesser von 0,2-0,6 mm haben.

27. Arzneimittelzubereitung nach Anspruch 21, dadurch gekennzeichnet, daß der Wirkstoff ein Wirkstoff aus der Gruppe Terpenaminoätherderivate, Antiemetica, insbeson dere basisch substituierte Benzamidderivate, Choleretica, insbesondere Trithionderivate, Chinolinderivate, Spasmolytica, insbesondere Atropin- und Scopolaminderivate, Phenylpropanolaminderivate, Antibiotica und Enzyme ist.

28. Arzneimittelzubereitung nach Anspruch 27, dadurch gekennzeichnet, daß der Wirkstoff ein Pinaveriumsalz ist.

29. Arzneimittelzubereitung nach Anspruch 28, dadurch gekennzeichnet, daß der Wirkstoff Pinaveriumbromid ist.

30. Arzneimittelzubereitung nach Anspruch 21, dadurch gekennzeichnet, daß als in Wasser nur bei pH-Werten von $\leq$ 5 lösliche polymere Filmbilder Mischpolymerisate aus basisch substituierten Methacrylsäureestern und neutralen Methacrylsäureestern verwendet werden.

31. Arzneimittelzubereitung nach Anspruch 30, dadurch gekennzeichnet, daß der Filmbildner ein Mischpolymerisat aus Dimethylaminoäthylmethacrylat und neutralen Methacrylsäureestern mit einem mittleren Molekulargewicht zwischen 100.000 und 200.000 ist.

32. Arzneimittelzubereitung nach Anspruch 21, dadurch gekennzeichnet, daß als in Wasser unlösliche polymere Filmbildner wasserunlösliche Zellulosederivate oder Schellack verwendet werden.

33. Arzneimittelzubereitung nach Anspruch 32, dadurch gekennzeichnet, daß das wasserunlösliche Zellulosederivat eine Äthylzellulose ist.

34. Arzneimittelzubereitung nach Anspruch 21, dadurch gekennzeichnet, daß in dem Film ein Anteil von 20-40 % des Gesamtgewichtes des Filmes aus in Wasser nur bei pH-Werten $\leq$5 löslichen polymeren Filmbildnern besteht.

35. Arzneimittelzubereitung nach Anspruch 21, dadurch gekennzeichnet, daß in dem Film ein Gewichtsverhältnis von in Wasser nur bei pH-Werten $\leq$5 löslichen polymeren Filmbildnern zu in Wasser unlöslichen polymeren Filmbildnern von 0,3:1 bis 2:1 vorliegt.

36. Arzneimittelzubereitung nach Anspruch 21, dadurch gekennzeichnet, daß der Film zwischen 12 und 25 % seines Gesamtgewichtes an wasserabweisenden Hilfsstoffen enthält.

37. Arzneimittelzubereitung nach Anspruch 35, dadurch gekennzeichnet, daß in dem Film ein nur bei pH-Werten $\leq$5 in Wasser lösliches Copolymerisat aus Dimethylaminoäthylmethacrylat und neutralen Methacrylsäureestern mit einem mittleren Molekulargewicht zwischen 100.000 und 200.000 und eine in Wasser unlösliche Athylzellulose und/oder Schellack vorliegen.

38. Arzneimittelzubereitung nach Anspruch 37, dadurch gekennzeichnet, daß der Film 12-25 % seines Gesamtgewichtes an wasserabweisenden Hilfsstoffen enthält und daß das Acrylatcopolymerisat und die Athylzellulose und/oder Schellack in einem Gewichtsverhältnis von 0,8:1 bis 2:1 vorliegen.

39. Arzneimittelzubereitung nach Anspruch 27, dadurch gekennzeichnet, daß der Wirkstoff Metoclopramid ist.

40. Arzneimittelzubereitung nach Anspruch 27, dadurch gekennzeichnet, daß der Wirkstoff Salbutamol ist.